# EUROPEAN PATENT SPECIFICATION

(11) **EP 1 777 208 B1**
(45) Date of publication and mention of the grant of the patent: **03.02.2010**
(21) Application number: 05022864.2
(22) Date of filing: 20.10.2005
(51) Int. Cl.: C07C 2/08, C07C 7/09

(54) **Method for oligomerization of ethylene and reactor system therefore with cooling device**
Verfahren zur Oligomerisierung von Ethylen und Reaktorsystem dafür mit Kühlvorrichtung
Procédé d'oligomérisation d'éthylène et réacteur à cet effet avec dispositif de refroidissement

(43) Date of publication of application: 25.04.2007
(73) Proprietor: Saudi Basic Industries Corporation, Riyadh 11422 (SA); Linde AG, 80331 München (DE)
(72) Inventor: Schneider, Richard, 82449 Uffing (DE); Fritz, Peter M., 82008 Unterhaching (DE); Muschelknantz, Sebastian, 81479 München (DE); Bölt, Heinz, 82515 Wolfratshausen (DE); Ali, Talal, 11422 Riyadh (SA); Mousa, Fuad, 11422 Riyadh (SA)
(74) Representative: Winkler, Andreas Fritz Ernst

(56) References cited:
- DE-C1- 4 338 414
- US-A- 4 242 531

## Description

The present invention relates to a method for oligomerisation of ethylene to form linear alpha-olefins in an oligomerisation reactor in the presence of solvent and catalyst, wherein a reaction product containing unreacted ethylene and light linear alpha-olefins is discharged from the reactor and passed to at least one first direct cooling device for separating the reaction product into an ethylene rich gaseous fraction and a light linear alpha-olefins richt liquid fraction, and to a reactor system therefore.

Methods for oligomerisation of ethylene are widely known in the art. For example, DE 43 38 414 C1 discloses a process for the preparation of linear alpha-olefins by oligomerisation of ethylene, wherein oligomerisation takes place in the presence of an organic solvent and a homogenous liquid catalyst. Usually, a catalyst is utilized in that process comprising a zirconium component and an organoaluminum component which acts as a co-catalyst. The oligomerisation may be carried out in that starting material comprising monomer, catalyst, co-catalyst and solvent is transferred to a reactor equipment. After conversion in the reactor equipment, product material comprising oligomers, non-reacted monomer, catalyst, co-catalyst and solvent may be discharged from the reactor equipment and can be further processed. According to DE 43 38 414 C1 a reaction product containing unreacted ethylene and light alpha-olefins may be discharged and transferred to a heat exchanger where the mixture is cooled to a specific temperature, for example 35°C. The liquid fraction obtained by cooling (containing substantially light alpha-olefins) is re-introduced into the reactor. The gaseous fraction obtained (substantially containing unreacted ethylene) is transferred to another heat exchanger where the temperature is further decreased, for example to a temperature of about 5°C. In the second heat exchanger unreacted ethylene is substantially separated from remaining light alpha-olefins. The separated alpha-olefins may then be further processed, for example in a rectification column.

Thus, in prior art methods for oligomerisation of ethylene, relatively small amounts of light linear alpha-olefins are generally separated from a relatively large gaseous ethylene stream by partial condensation in several heat exchangers. As the heat-transfer co-efficients of the gaseous components are relatively small, the heat exchangers utilized are relatively large and must be supported in complicated constructions, such as steel constructions.

It is an object of the present invention to provide a method for oligomerisation of ethylene which overcomes the drawbacks of the prior art. Especially a method shall be provided which may utilize smaller heat exchangers which do not have to be incorporated in complicated constructions.

Additionally, it is an object of the present invention to provide a reactor system for carrying out the inventive method.

The first object is achieved in that at least a portion of the light linear alpha-olefins rich liquid fraction is passed to a second cooling device for lowering the temperature thereof and is subsequently re-introduced into the direct cooling device.

Preferably, re-introduction is at the top of the direct cooling device.

In addition it is also preferred that unreacted ethylene is recycled into the oligomerisation reactor or further processed.

In a preferred embodiment another portion of the light alpha-olefins rich liquid fraction is transferred to a separation device.

Preferably, the separation device is a rectification column.

In addition it is also preferred, that the first direct cooling device contains random packing, structured packing and/or trays.

In one aspect to the light alpha-olefins rich liquid fraction being re-introduced into the first direct cooling device an additional solvent is added.

In a preferred embodiment the additional solvent is a heavier alpha-olefins fraction, preferably also obtained in the oligomersation method.

In one aspecet, the light alpha-olefins are C₄-C₈-olefins and/or heavy alpha-olefins are C₁₀-C₁₈-olefins

In addition it is also preferred that the light alpha-olefins rich fraction is cooled in the second cooling device to a temperature of about 3°C. The mixture of ethylene and light linear alpha-olefins is cooled in the first direct cooling device preferably to a temperature of about 8°C.

The second object is achieved by a reactor system for oligomerisation of ethylene to form linear alpha-olefins, preferably utilizing an inventive method, comprising an oligomerisation reactor and a separation unit connected thereto for separating unreacted ethylene from light alpha-olefins, the separation unit comprising at least one direct cooling device having a loopline for light alpha-olefins passing through a second cooling device.

According to the present invention, the heat is advantageously removed from liquid stream (light linear alpha olefins) instead of a gaseous stream as done so far in the prior art. In fact, the heat is removed from the loop cycling light liquid alpha-olefins. Thereby, substantially higher heat-transfer coefficients may be realized. Thus, the heat exchangers and cooling devices utilized in the method of the present invention may be designed substantially smaller and do not need to be integrated in complicated constructions. The method of the present invention additionally provides an increased separation efficiency, since in the direct cooling device more than one theoretical separation stage can be applied.

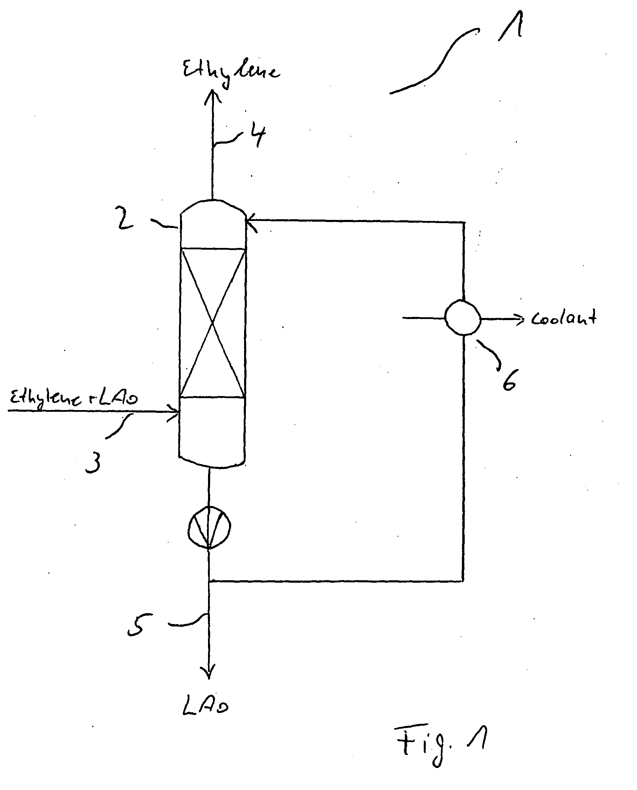

Additional features and advantages of the inventive method and reactor system are further illustrated with reference to the accompanying drawing, wherein figure 1 illustrates a schematic diagram of a separation unit which form part of the inventive reactor system.

Figure 1 illustrates a separation unit 1 comprising a first direct cooling device 2. From an oligomerisation reactor (not shown) unreacted ethylene and light linear alpha-olefins are transferred into the first direct cooling device 2 via line 3 directly or indirectly. In the first direct cooling device 2, the mixture of unreacted ethylene and light linear alpha-olefins is cooled to a specific temperature, for example 8°C, so that the reaction product is separated into an ethylene rich gaseous fraction and an light alpha-olefins rich liquid fraction. The ethylene rich fraction may be discharged from the first direct cooling device 2 via line 4 and may be re-introduced into the oligomerisation reactor. The light alpha-olefins rich liquid fraction may be also discharged from the first direct cooling device 2 via line 5. At least a portion of that fraction may, however, be passed to a second cooling device 6, preferably utilizing an adequate coolant, where the light alpha-olefin rich liquid fraction is further cooled, for example to a temperature of about 3°C. The thus cooled light alpha-olefins rich liquid fraction is then re-introduced into the first direct cooling device 2, preferably at the top thereof. Thus, the re-introduced light alpha-olefins fraction may be utilized as washing agent to reduce further the amount of linear alpha-olefins present in the gaseous stream of unreacted ethylene.

As the heat of the reaction products is removed according to the present invention from a liquid stream instead of a gas stream, the sizes of involved heat exchangers or cooling device may be significantly reduced. This may result in cost and energy savings.

Another portion of the light alpha-olefins rich fraction which is not cooled and re-introduced into the first direct cooling device 2, may be transferred to a separation device (not shown) such as a rectification column to fractionate the linear alpha-olefins obtained.

## Claims

1. Method for oligomerisation of ethylene to form linear alpha-olefins in an oligomerisation reactor in the presence of solvent and catalyst, wherein a reaction product containing unreacted ethylene and light linear alpha-olefins is discharged from the oligomerisation reactor and passed to at least one first direct cooling device (2) for separating the reaction product into an ethylene rich gaseous fraction and a light linear alpha-olefins rich liquid fraction, **characterized in that** at least a portion of the light linear alpha-olefins rich liquid fraction obtained is passed to a second cooling device (6) for lowering the temperature thereof and is subsequently re-introduced into the first direct cooling device (2).

2. Method according to claim 1, wherein re-introduction is at the top of the direct cooling device (2).

3. Method according to claim 1 or 2, wherein unreacted ethylene is recycled into the oligomerisation reactor or further processed.

4. Method according to any of the preceding claims, wherein another portion of the light alpha-olefins rich liquid fraction is transferred to a separation device.

5. Method according to claim 4, wherein the separation device is a rectification column.

6. Method according to any of the preceding claims, wherein the first direct cooling device (2) contains random packing, structured packing and/or trays.

7. Method according to any of the preceding claims, wherein to the light alpha-olefin rich liquid fraction being re-introduced into the first direct cooling device (2) an additional solvent is added.

8. Method according to claim 7, wherein the additional solvent is a heavier alpha-olefins fraction, preferably also obtained in the oligomerisation method.

9. Method according to any of the preceding claims, wherein light alpha-olefins are C₄-C₈-olefins and/or heavy alpha-olefins are C₁₀-C₁₈-olefins.

10. Method according to any of the preceding claims, wherein the light alpha-olefin rich fraction is cooled in the second cooling device (6) to a temperature of about 3°C.

11. Reactor system for oligomerisation of ethylene to form linear alpha-olefins, preferably utilizing a method according to any of the preceding claims 1 to 10, comprising an oligomerisation reactor and a separation unit connected thereto for separating unreacted ethylene from light alpha-olefins, the separation unit comprising at least one direct cooling device (2) having a loopline for light alpha-olefins passing through a second cooling device (6).

## Patentansprüche

1. Verfahren zur Oligomerisation von Ethylen, um lineare α-Olefine in einem Oligomerisationsreaktor in der Gegenwart von Lösungsmittel und Katalysator zu bilden, wobei ein Reaktionsprodukt, das nicht umgesetztes Ethylen und leichte lineare α-Olefine enthält, aus dem Oligomerisationsreaktor abgeführt wird und zu wenigstens einer ersten direkten Kühlvorrichtung (2) zum Auftrennen des Reaktionsprodukts in eine ethylenreiche, gasförmige Fraktion und eine an leichten linearen α-Olefinen reiche flüssige Fraktion geführt wird, **dadurch gekennzeichnet, dass** wenigstens ein Teil der erhaltenen, an leichten linearen α-Olefinen reichen flüssigen Fraktion zu einer zweiten Kühlvorrichtung (6) zum Absenken der Temperatur derselben geführt wird und anschließend in die erste direkte Kühlvorrichtung (2) wieder eingeführt wird.

2. Verfahren nach Anspruch 1, wobei die Wiedereinführung am Kopfende der direkten Kühlvorrichtung (2) erfolgt.

3. Verfahren nach Anspruch 1 oder 2, wobei nicht umgesetztes Ethylen in den Oligomerisationsreaktor recycliert oder weiter verarbeitet wird.

4. Verfahren nach einem der vorangehenden Ansprüche, wobei ein anderer Teil der an leichten α-Olefinen reichen flüssigen Fraktion zu einer Auftrennvornchtung überführt wird.

5. Verfahren nach Anspruch 4, wobei die Auftrennvorrichtung eine Rektifikationssäule ist.

6. Verfahren nach einem der vorangehenden Ansprüche, wobei die erste direkte Kühlvorrichtung (2) eine statistische Packung, strukturierte Packung und/oder Böden enthält.

7. Verfahren nach einem der vorangehenden Ansprüche, wobei zu der an leichten α-Olefinen reichen, flüssigen Fraktion, die in die erste direkte Kühlvorrichtung (2) wiedereingeführt wird, ein zusätzliches Lösungsmittel zugegeben wird.

8. Verfahren nach Anspruch 7, wobei das zusätzliche Lösungsmittel eine schwerere α-Olefin-Fraktion ist, die bevorzugt ebenfalls im Oligomerisationsverfahren erhalten wird.

9. Verfahren nach einem der vorangehenden Ansprüche, wobei leichte α-Olefine C₄-C₈-Olefine sind und/oder schwere α-Olefine C₁₀-C₁₈-Olefine sind.

10. Verfahren nach einem der vorangehenden Ansprüche, wobei die an leichten α-Olefinen reiche Fraktion in der zweiten Kühlvorrichtung (6) auf eine Temperatur von etwa 3°C gekühlt wird.

11. Reaktorsystem zur Oligomerisation von Ethylen, um lineare α-Olefine zu bilden, bevorzugt einsetzend ein Verfahren nach einem der vorangehenden Ansprüche 1 bis 10, umfassend einen Oligomerisationsreaktor und eine Abtrenneinheit, die daran zum Abtrennen nicht umgesetzten Ethylens von leichten α-Olefinen angefügt ist, wobei die Abtrenneinheit wenigstens eine direkte Kühlvorrichtung (2) mit einer Kreislaufleitung für leichte α-Olefine, die durch eine zweite Kühlvorrichtung (6) führt, umfasst.

## Revendications

1. Procédé d'oligomérisation de l'éthylène pour former des alpha-oléfines linéaires dans un réacteur d'oligomérisation en présence d'un solvant et d'un catalyseur, dans lequel un produit de réaction contenant de l'éthylène n'ayant pas réagi et des alpha-oléfines linéaires légères est évacué du réacteur d'oligomérisation et est envoyé dans au moins un premier dispositif de refroidissement direct (2) pour séparer le produit de réaction en une fraction gazeuse riche en éthylène et une fraction liquide riche en alpha-oléfines linéaires légères, **caractérisé en ce qu'**au moins une partie de la fraction liquide riche en alpha-oléfines linéaires légères est envoyée dans un deuxième dispositif de refroidissement (6) pour abaisser sa température et est ultérieurement ré-introduite dans le premier dispositif de refroidissement direct (2).

2. Procédé selon la revendication 1, dans lequel la réintroduction se fait par le haut du dispositif de refroidissement direct (2).

3. Procédé selon la revendication 1 ou 2, dans lequel l'éthylène n'ayant pas réagi est recyclé dans le réacteur d'oligomérisation ou re-traité.

4. Procédé selon l'une quelconque des revendications précédentes, dans lequel une autre partie de la fraction liquide riche en alpha-oléfines légères est transférée dans un dispositif de séparation.

5. Procédé selon la revendication 4, dans lequel le dispositif de séparation est une colonne de rectification.

6. Procédé selon l'une quelconque des revendications précédentes, dans lequel le premier dispositif de refroidissement direct (2) contient un garnissage aléatoire, un garnissage structuré et/ou des plateaux.

7. Procédé selon l'une quelconque des revendications précédentes, dans lequel un solvant additionnel est ajouté à la fraction liquide riche en alpha-oléfines légères qui est ré-introduite dans le premier dispositif de refroidissement direct (2).

8. Procédé selon la revendication 7, dans lequel le solvant additionnel est une fraction d'alpha-oléfines plus lourdes, de préférence également obtenues dans le procédé d'oligomérisation.

9. Procédé selon l'une quelconque des revendications précédentes, dans lequel les alpha-oléfines légères sont des oléfines en C₄-C₈ et/ou les alpha-oléfines lourdes sont des oléfines en C₁₀-C₁₈.

10. Procédé selon l'une quelconque des revendications précédentes, dans lequel la fraction riche en alpha-oléfines légères est refroidie dans le deuxième dispositif de refroidissement (6) à une température d'environ 3 °C.

11. Système de réacteur pour l'oligomérisation de l'éthylène dans le but de former des alpha-oléfines linéaires, de préférence en utilisant un procédé selon l'une quelconque des revendications 1 à 10, comprenant un réacteur d'oligomérisation et une unité de séparation raccordée à celui-ci pour séparer l'éthylène n'ayant pas réagi des alpha-oléfines légères, l'unité de séparation comprenant au moins un dispositif de refroidissement direct (2) ayant un circuit fermé pour que les alpha-oléfines légères passent à travers un deuxième dispositif de refroidissement (6).
